# EUROPEAN PATENT APPLICATION

(11) **EP 1 275 442 A1**
(43) Date of publication of application: **15.01.2003**
(21) Application number: 01202695.1
(22) Date of filing: 13.07.2001
(51) Int. Cl.: B05D 1/04, A61C 8/00, A61L 27/32

(54) **Electrostatic spray deposition (ESD) of biocompatible coatings on metallic substrates**

(71) Applicant: Stichting voor de Technische Wetenschappen, 3502 GA Utrecht (NL)
(72) Inventor: Jansen, Johannes Arnoldus, 6662 BM Elst (NL); Schoonman, Johannes, 2242 JH Wassenaar (NL); Leeuwenburgh, Sander Cornelis Gerardus, 6532 CZ Nijmegen (NL)
(74) Representative: Jorritsma, Ruurd

(57) **Abstract**

The invention relates to a method for depositing a coating onto an implant for implantation in bone. In this method a coating is deposited by the ESD technique which can be described as forcing a precursor solution through a capillary which is subjected to an electrical field. Of particular relevance for bone implants are coatings which comprise calcium and phosphate. The invention also relates to implants having a coating which is deposited by this method. A particular example of such an implant is a dental implant for the fixation of a dental prosthesis.

## Description

### FIELD OF THE INVENTION

The invention relates to a method for depositing a coating onto an implant for implantation in bone. A particular example of such an implant is a dental implant

### BACKGROUND OF THE INVENTION

Implants have a function as fixation elements in bone or serve as anchoring members of orthopaedic and dental prostheses. In order to successfully allow implants to exert their function it has previously been proposed to deposit a coating of biocompatible material onto the implant to promote fixation of the implant to the bone. A suitable biocompatible material may comprise calcium phosphate. In particular implants may be coated with hydroxyapatite (Ca₅(PO₄)₃OH) as this is the main constituent of bone.

Various techniques are known for the deposition of hydroxyapatite onto implants including electrophoretic deposition, immersion coating, hot isostatic pressing and plasma spraying. Plasma spraying is the most widely used technique for biomedical applications. Disadvantages of the plasma spraying technique are that relatively thick layers of at least 50 µm need to be formed. Consequently the coated surface may not have the same surface geometry as the underlying implant. Moreover, the sprayed coating may lack mechanical strength. The layer is brittle and can easily break off.

Developments to overcome the disadvantages of the plasma spraying technique have focussed on sputtering techniques to deposit thin layers of coating material onto implants. Examples of such sputtering techniques are ion beam sputtering as described in US 4,944,754 and the plasma sputtering process as described in US 5,543,019. Although using these techniques successfully thin hydroxyapatite coatings have been deposited onto implants there are several disadvantages associated with sputtering techniques. Firstly the sputtering technique requires complex and costly apparatus as high vacuums are required. The most important disadvantage however is that with the sputtering technique the morphology of deposited coatings cannot be controlled. It is only possible to deposit entirely dense coatings. Successful integration of a coated implant in bone tissue does not only depend on the fysico-chemical natureof the coated material. It also depends on the microstructure and the roughness of the coating surface.

### SUMMARY OF THE INVENTION

It is an object of the present invention to provide implants having improved coatings for successful integration into bone tissue.

It has now surprisingly been found that using the Electrostatic Spray Deposition (ESD) technique a coating of biocompatible material for bone onto an implant can be deposited. Advantageously the morphology of the coating can be controlled by varying the parameters of the ESD process.

In essence the ESD technique is forcing a liquid through a capillary which is subjected to an electrical field upon which the liquid leaves the capillary in the form of a spray the shape of which is determined by the electrical field. The liquid is a solution of one or more precursors of which the coating on an object should be formed. By placing an object in the spray that is formed the precursor solution deposits onto the object.

Thus the invention relates to a method for depositing a coating onto an implant for implantation in bone comprising the steps of forcing a precursor solution through a capillary which is subjected to an electrical field and placing the implant in the area of the spray that leaves the outlet of the capillary.

The invention also relates to implants for implantation in bone having a coating which is deposited with ESD.

### DETAILED DESCRIPTION OF THE INVENTION

Electrostatic Spray Deposition is a relatively recently developed technique. In particular it has been developed for the preparation of thin-film components for rechargeable lithium-ion batteries and for coating solid oxide fuel cells with composite electrolyte thin films (Chen, 1998, PhD thesis Technical University Delft: Thin film components for lithium-ion batteries).

In short the technique can be described as a liquid being forced, usually by means of a pump, through a capillary in the form of a metal nozzle which is subjected to an electric field. In doing so the liquid will leave the outlet of the nozzle in a spray of a particular form depending on the underlying electrohydrodynamic mechanism. When an object is positioned in line with the nozzle outlet the liquid leaving the nozzle will deposit onto the object.

The formation of a spray by an electric field is a process whereby a liquid jet breaks up into droplets under the influence of electrical forces. Depending on the strength of the electric field and the kinetic energy of liquid leaving the nozzle, different shapes of sprays will be obtained.

At the lowest liquid flow rates and a low electric field microdripping occurs. The applied electric field generates a surface charge in the droplet, resulting in an electric stress which reduces the effective surface tension stress in the droplet surface. A small droplet will be ejected. The large droplet at the capillary relaxes to its original shape. It will start to grow until a new droplet is ejected.

At a higher liquid flow rate and at a higher electric field than microdripping the cone-jet mode occurs. In contrast to the discontinuous droplet generation the cone-jet produces an aerosol with a continuous flow at. Increasing the applied potential difference will result into an increased electric field strength around the liquid cone. At a certain point, the liquid cone does not relax to a droplet shape anymore and droplet production becomes stable in time. This is the cone-jet mode. With increasing potential difference, the liquid cone of the cone-jet mode becomes smaller and smaller. At a certain moment, the liquid cone is too small for the capillary. The cone moves from the center of the capillary towards the edge. When the potential difference increases further, a second cone will appear; the so-called duo-jet mode. With increasing potential difference, more and more cones will be formed. For a multiple-jet mode, the bases of these cones are still attached. Together, these cones can be considered as one droplet with multiple spraying points. Also a large number of very small cones can be formed in a thin layer of liquid on the edge of the capillary this is called the rim-emission mode.

If the liquid flow rate is relatively high, then the kinetic energy of the liquid leaving the capillary can result in the formation of a long, free jet, which breaks up into droplets. This is the simple-jet mode. By increasing the electric field around this long jet, small jets can laterally emerge from the surface of the main jet. These jets break up into a polydisperse spray. This mode is called the ramified-jet mode.

According to the invention preferably the duo-jet mode was used. The liquid cones in the duo-jet mode are relatively small as a relatively large potential difference is applied over the electrodes. In the duo-jet mode, the current per spraying point is relatively large compared to the situation with the same nozzle with only one large cone attached. This means that the size distribution of a spray produced in the duo-jet mode is most likely somewhat wider than for a spray produced in the cone-jet mode using the same nozzle.

For the purpose of the ESD technique the liquid is a solution of one or more component(s) which the coating on the object should comprise. The latter components are referred to as precursors and a solution thereof is referred to as a precursor solution. When deposited on an object, upon evaporation of the solvent component of the precursor solution, a coating of precursor component(s) of the liquid will remain on the object. As set out in the examples several parameters play a role in the ESD process and influence the end result, i.e. the coating on an object.

According to the present invention it has been found that the Electrostatic Spray Deposition technique is suitable for depositing a coating onto implants for implantation in bone.

Compared to known methods to deposit films comprising calcium and phosphate advantageously the method of the invention allows the formation of other morphologies than only a dense coating. In particular the morphology of the deposited coating can be controlled by selection of the ESD parameters. An additional advantage is that compared to the dense coatings that can be deposited according to the state of the art the invention makes the deposition of porous coatings possible.

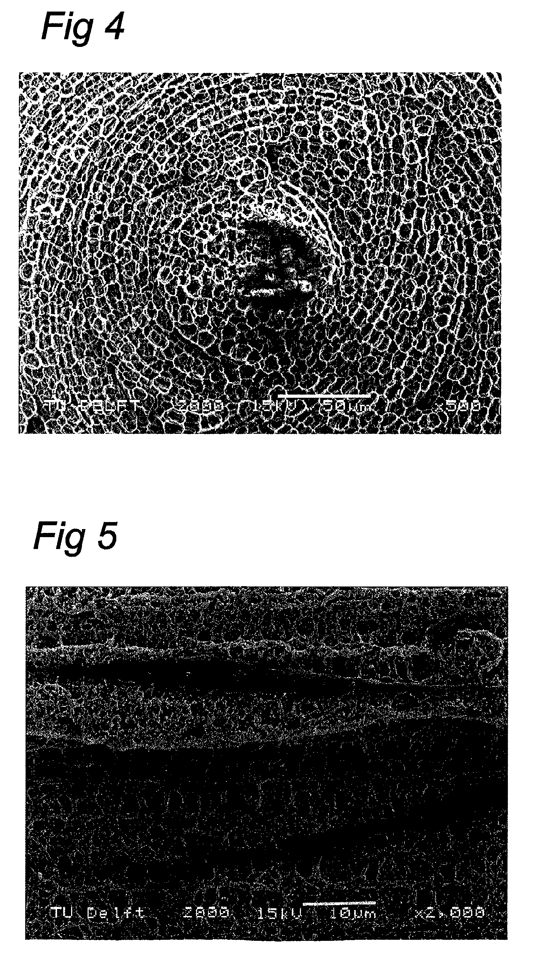

Another advantage is that the topography of the surface of the object that is coated is reflected in the coating morphology. This allows the preparation of custom made coatings on a specifically prepared surface The examples show stable attachment of cells and subsequent abundant cell growth on coatings that are deposited with the method of the invention. In addition, in particular in figure 5, the influence of the surface morphology on the attachment of cells is shown.

In one embodiment of the invention the deposition of a coating comprising calcium and phosphate is preferred as the coating is preferably biocompatible with bone tissue and calcium and phosphate are the main constituents of bone tissue. The coating comprises for instance calcium phosphate (CaHPO₄), monocalcium phosphate (Ca(H₂PO₄)₂), tricalcium phosphate (Ca₃(PO₄)₂), tetracalcium phosphate (CaO.Ca₃(PO₄)₂) or octacalcium phosphate (Ca₈H₂(PO₄)₆.5H₂O). The most abundant form in which calcium and phosphate are present is in bone tissue in the form of apatites. Apatites have the general formula Ca₅(PO₄)₃X wherein X is a suitable anion such as for instance OH, CO₃, F, Cl or NO₃. Preferably X is OH or CO₃ which results in apatites that are called hydroxyapatite (Ca₅(PO₄)₃(OH)) and carbonate apatite (Ca₅(PO₄)ₓ(CO₃)_{y}) respectively.

Thus according to the invention the precursor solution comprises calcium and phosphate. In this respect comprising calcium means comprising Ca²⁺ ions and comprising phosphate means comprising PO₄³⁻ ions, which includes H₃PO₄, H₂PO₄⁻ and HPO₄²⁻.

The molar ratio of calcium to phosphate in the precursor solution determines the composition of the coating. The coatings preferably have a molar ratio calcium to phosphate in the range of 0.5 to 2.0. A ratio of 1.5 corresponds to tricalcium phosphate and a ratio of 2.0 corresponds to tetracalcium phosphate. For a coating comprising hydroxyapatite a ratio of calcium to phosphate 1.67 is used. When a higher ratio, in particular a ratio of 1.80, is used the formation of a coating comprising carbonate apatite is favoured. The relative amount of each precursor that is required in a precursor solution can be determined by the skilled person depending on the composition of the coating that is required.

Thus in one embodiment according to the invention the molar ratio of calcium to phosphate in the precursor solution is in the range of about 0.5 to about 2, preferably is in the range of about 1.5 to about 1.8, more preferably is in the range of about 1.67 to about 1.8, even more preferably is about 1.67.

Suitable precursor sources for calcium and phosphate to prepare a precursor solution are calcium nitrate (Ca(NO₃)₂.4H₂O) and phosphoric acid (H₃PO₄). It is well within the reach of the skilled person to find alternative sources for calcium and phosphate. The absolute concentration of precursors sources depends on the solubility of the specific precursor source in the solvent that is used in the ESD process. The concentration of calcium nitrate is preferably in the range 0.001-0.025 M. Consequently the concentration of phosphoric acid is in the range 0.0005-0.050 M.

In another embodiment the coating further comprises glass-forming components. Such components have an advantageous effect on the adhesion strength of the coating to the implant. A coating comprising glass-forming components is for instance 45S5 Bioglass®, which has the following composition: SiO₂ 45.0 weight%, P₂O₅ 6.0 weight%, CaO 24.4 weight% and Na₂O 24.5 weight%. The skilled person will be able to determine different compositions which will form a glass under ESD conditions. In order to deposit a coating comprising glass-forming components with the ESD technique besides for calcium and phosphate suitable precursors for silicium and sodium have to be used. Suitable precursors are for instance tetramethyl ortho-silicate ((CH₃)₄O₄Si) and sodium hydroxide (NaOH). Depending on the composition of the components in the coating the skilled person will be able to determine the relative amounts of precursors in a precursor solution that is required.

In a further embodiment the precursor solution can comprise additives, such as water (preferably 1-5 vol%) or nitric acid (HNO₃ 65% 0.25-1vol%), which influence the morphology of the coating.

The ESD technique also allows the deposition of (poly)peptides or proteins onto a substrate surface. In a further embodiment the precursor solution can also comprise elements which support cell growth such as for instance bone-growth supporting proteins.

The precursor preferably dissolves well in the solvent of the precursor solution. Precipitation of precursor in the solvent should be avoided to prevent clogging of the nozzle in the ESD apparatus. The boiling point of the solvent is an important parameter for control of the morphology of the coating.

According to the invention preferably the solvent is an alcohol. Typically, depending on the desired morphology, an alcohol with a low boiling point or an alcohol with a high boiling point or a mixture thereof is used. As a consequence of the temperature to which an implant during the coating process is preferably heated (see below), preferably the solvent has a boiling point lower than 450°C. More preferably the boiling point of the solvent is in the range of 50°C-250°C. Ethanol, boiling point 78°C, and butyl carbitol (other names for butyl carbitol are 2-(2-butoxyethoxy)ethanol and di(ethylene glycol butyl ether), boiling point 231°C, or a mixture of the two are suitable solvents according to the invention, however, other alcohols or mixtures of alcohols may be used as well.

For the implant to be coated using the ESD technique suitably the implant consists of metal or at least at the side to be coated comprises a metal surface. In one embodiment according to the invention suitable metals for implants are niobium, tantalum, cobalt-chromium alloys, (stainless) steel and in particular titanium and titanium alloys. Several metals, such as for instance titanium, will have an oxide layer at the surface due their inherent properties and natural appearance. Another possibly suitable material to be coated is aluminium oxide (alumina-ceramic; Al₂O₃).

According to the invention preferably the implant that is coated is heated. This influences the evaporation of the solvent of the precursor solution and consequently influences the precipitation process and thus ultimately influences the morphology of the coating. Depending on the desired morphology a particular temperature of heating is selected. Preferably the implant is heated to a temperature in the range of 250°C to 450°C.

When a coating is deposited onto an implant with the ESD technique the coated implant can be subjected to a heat treatment. To prevent oxidation of the material of the implant the heat treatment should be short, preferably in the range of 5-30 s, and is preferably carried out using infrared radiation. Such a heat treatment influences the crystallinity of the coating. Heat treatment at higher temperature results in a more 'crystalline' coating, whereas a lower temperature results in a more 'amorphous' coating.

Also the heat treatment influences the composition of the coating as it induces the formation of hydroxyapatite. Crystallinity and composition of the coating influence the attachment of cells to the coating. Depending on the particular purpose the skilled person will be able to select a particular composition and crystallinity to suit that purpose.

Thus in another embodiment the method according to the invention comprises the step of heating the coated implant to a temperature in the range of 450-1250°C, preferably by infrared radiation for 5-30 s.

With ESD a thin coating can be deposited which is less than 15 µm thick, preferably is in the range of 0.5-10 µm, more preferably is in the range of 1-5 µm thick. A parameter that is of influence on the thickness of the coating is the time during which a coating is deposited onto an implant. Usually coating is carried in less than 2 hrs and usually for more than 7.5 mins. A suitable distance between the outlet of the nozzle and the implant is in the range of 1-5 cm, preferably 2.5-3.5 cm.

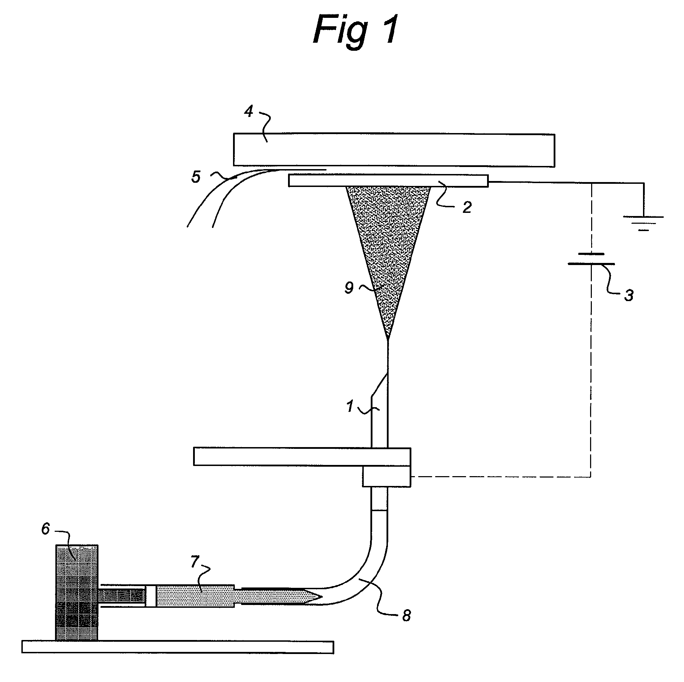

Compared to apparatus that is required to deposit plasma sputtered coatings the ESD apparatus is simple, inexpensive and easy to handle. Figure 1 shows schematically the relevant apparatus. In this figure the apparatus operates in a vertical position, spraying upwards. Other positions, for instance horizontal, can however just as well be used. It is also possible to use a vertical set-up with the spray directed downwards. The size and type of nozzle has an effect on the spraying cone and can be varied.

The geometry of the nozzle also has a prominent influence on the shape of the spray. The outlet of the nozzle can be flat or the tip of the nozzle can end in an angle, which is called a tilted outlet. For instance tilted outlets of 15° or 30° or even higher values can be used. Also the diameter of the nozzle can be varied. In the set-up described in the examples the nozzle has a tilted outlet of 30° and an inner and an outer diameter of 0.6 and 0.8 mm respectively and. Thus typically the diameter of the nozzle is in the milimeter range.

Within the set of parameters, as described in the examples, that influence the ESD process the flow rate of the liquid that is pumped through the capillary and the applied electric field play a prominent role. According to the invention the flow rate is in the range of 0.2-5 ml/h, preferably in the range of 1-3 ml/h. The strength of the electric field is preferably in the kV-range, more preferably in the range of 4-12 kV and even more preferably in the range of 6-9 kV. When using a nozzle with a geometry that substantially differs from the nozzle used in the examples the preferred values for the parameters given above may have to be adjusted. If such is the case it is well within the reach of the skilled person to experimentally determine proper adjustments and arrive at a suitable set of values for all parameters.

Compared to sputter deposition ESD has a high deposition efficiency and the technique is relatively clean. For coating larger objects the object can be moved from position to position in the spraying cone. Also an array of nozzles can be used. Also it is possible to let a large object or a large number of small objects pass on a conveyor belt a nozzle or an array of nozzles.

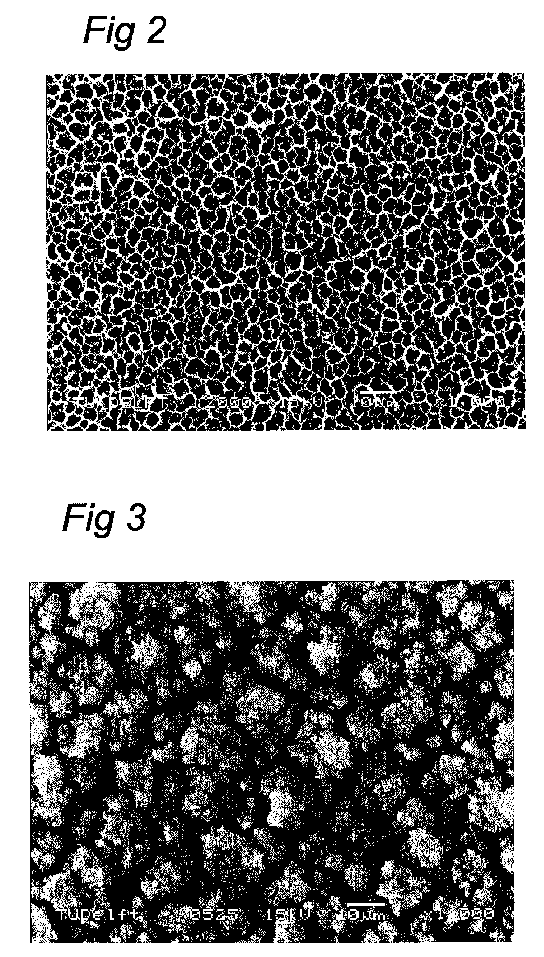

By proper selection of the ESD parameters the morphology of the coating can be controlled and can be varied from a dense coating to a granular coating which in the examples is called a broccoli coating, to even a porous coating with reticular, interconnected porosity, which in the examples is called a sponge coating. The porous nature of the sponge coatings is exemplified in figures 2, 4, 5 and 7. Scanning Electron Microscopy (SEM) is a suitable technique to study the structure and thus the porosity of the coating. SEM also allows the size of the pores to be determined.

In general any implant of which at least a part is in contact with bone tissue is suitable to be coated according to the method of the invention. Examples of such implants are pins, plates and screws to be used for fixation in case of bone fractures or other surgical procedures but also joints such as hips and knees, or parts of joints onto which preferably bone should settle can be suitably coated according to the method of the invention. Of particular interest are dental implants in the form of screws for the fixation of dental prostheses, crowns and bridges.

The invention also relates to implants for implantation in bone provided with a coating obtainable by the method according to the invention. In particular the sponge coating displays advantageous properties for the attachment of cells. Thus in a preferred embodiment the implant has a porous coating comprising calcium and phosphate. Preferably the coating has pores in the range of 0,1-25 µm, more preferably in the range of 0,5-15 µm and even more preferably in the range of 0.8-10 µm. In a preferred aspect the invention relates to dental implants.

### DESCRIPTION OF THE FIGURES

**Figure 1:** ESD set-up in vertical configuration.
**Figure 2:** Scanning electron micrograph of an ESD derived sponge coating.
**Figure 3:** Scanning electron micrograph of an ESD derived broccoli coating.
**Figure 4:** Scanning electron micrograph showing the influence of substrate topography on the morphology of ESD derived sponge coating for a machined cp-Ti substrate.
**Figure 5:** Scanning electron micrograph of RBM cells attached to sponge ESD-coatings after 2 days in culture.
**Figure 6:** Scanning electron micrograph of broccoli ESD-coatings after 7 days of RBM cell culture.
**Figure 7:** Scanning electron micrograph of sponge ESD-coatings after 7 days of RBM cell culture.

### EXAMPLES

### ESD-equipment:

A vertical ESD set-up (ESD-ACT-XY03, TU Delft) has been used in this study to deposit inorganic coatings. Figure 1 gives a schematic view of the set-up, which is operated in a fume hood.
In this unit, the spray is directed upwards from the nozzle (1) to the substrate holder (2). The set-up consists mainly of the following parts:
(i) An electrospraying unit, including a high DC voltage power supply (3) (Fug, HCN14-20000) capable of providing voltages up to 20 kV, a hollow nozzle (1) made of RVS 304 stainless steel (< 0.08 wt% C, 17.5 - 19.0 wt% Cr, 9 - 11 wt% Ni) with an outer diameter smaller than 1.00 mm, an RVS 304 substrate holder (2). Preferably the substrate holder is mounted on an x-y table (not shown), which motion can be controlled by a computer. The unit may comprise a high-intensity halogen lamp (not shown), which enables the visualization of the spraying cone (9). For safety reasons the substrate holder is preferably grounded.
(ii) A temperature controlling unit (Eurotherm Controls model 2216), including a heating element (4) and temperature controller (5).
(iii) A liquid feeding unit connected to a precursor liquid reservoir (not shown) including a syringe pump (6) (Kd Scientific 100-3113), a syringe (7) preferably of glass with a volume between 2.5 to 10 ml, and flexible tube (8) of a chemically resistant rubber, for instance Bioprene/Marprene®, inner and outer diameter 0.8 mm and 4.0 mm, respectively.
(iv) Supporting elements like electronic connection cables suitable for high-voltage operation, a digital ruler to measure the nozzle-to-substrate distance, and drain collection container (all not shown). This collection container is highly desirable in the vertical, upside-down spraying configuration in the case of liquid overflow.

### Deposition of ESD-coatings

The spray is directed towards the heated substrate holder, which contains for instance a commercially pure Ti substrate. For optimal deposition results, precursor solutions should be clear and unprecipitated without any precipitates that could act as nucleation sites for further precipitation. For reproducible production of homogeneous coatings and ensuring a proper process control preferably premature precipitation of the precursor solutions in the glass syringe, silicone tube or metal nozzle before generation of the spray should be avoided.
Therefore, fresh and clean precursor solutions are preferably used for each deposition. Before and after each deposition run, the syringe, silicone tube and nozzle are flushed with clean ethanol in order to remove possible deposits. For a more thorough cleaning of the nozzle, the nozzle can be cleaned ultrasonically in 10-15 vol% HNO₃ in ethanol every fifth hour of operation.
Inorganic and insulating material being left onto the substrate holder next to the mounted substrates can be removed every fifth hour of operation with 10-15 vol% HNO₃ in ethanol. Thereafter, the substrate holder is rinsed with pure ethanol. The formation of an insulating layer disturbs the homogeneous electric field between the conducting nozzle and substrate holder and thus the spraying process.
The substrate is cleaned ultrasonically in acetone (15 minutes) and ethanol (15 minutes) prior to the deposition.

### Nozzle geometry

The geometry of the nozzle affects the spraying mode. For instance nozzles with a flat outlet or with a tilted outlet, i.e. the tip of the nozzle ends in an angle of for instance 15° or 30°, can be used. The results described hereinbelow were obtained with a tilted nozzle having an apex angle of 30° and an inner and an outer diameter of 0.6 and 0.8 mm respectively.

### Precursor solutions

Ca(NO₃)₂.4H₂O (Merck) and H₃PO₄ (85 wt%, J.T. Baker) were used as precursors for calcium and phoshate, respectively.
Calcium nitrate was chosen for its higher solubility as compared to for example calcium acetate. With ESD, apolar, alcoholic solvents are used, in which salts do not dissolve easily. Moreover, nitrate ions are not incorporated into crystalline precipitated apatites.
Phosphoric acid was used as a source for phosphate ions, instead of the commonly used ammonium phosphate (NH₄)₂HPO₄²⁻ with added NH₄OH. This latter precursor seems not appropriate because of the fast precipitation of hydroxyapatite under the very alkaline reaction conditions. ESD is based on the atomization of clear solutions without any precipitation before the generation of the spray. The acidity of the precursor solutions should be higher than the alkaline reaction conditions of aqueous precipitation techniques.
Water was used in some precursor solutions to investigate the incorporation of hydroxyl groups into the apatite structure, if any. Also, HNO₃ (65%) was sometimes added to study its influence on the solute precipitation and spraying characteristics of the precursor solutions.

### Solvents

Ethanol (ET, C₂H₆O) and butyl carbitol (BC, C₈H₁₈O₃, 99%, Aldrich) were used as low- and high-boiling point solvents, respectively. Some physical and chemical data are given below. Butyl carbitol was used to prevent extensive solvent evaporation during spraying.

| Physical and chemical data of the solvents used | | |
|---|---|---|
| | **Ethanol (ET)** | **Butyl carbitol (BC)** |
| Molar weight | 46.07 | 162.23 |
| Melting point (°C) | -114 | -68 |
| Boiling point (°C) | 78 | 231 |
| Density (g/cm³) | 0.789 | 0.955 |
| Viscosity (mPas) | 1.074 | 4.76 |
| Surface tension (mN/m) | 21.97 | 30.0 |
| Dielectric constant ε | 24 | 10 |
| Solubility | miscible in H₂O and BC | miscible in H₂O and ET |

### Range of deposition parameters

In order to influence the spraying characteristics, the morphology and the chemical structure of the deposited thin calcium phosphate films, the following parameters can be varied:
(i) The applied voltage V [kV]; the potential difference can be varied between 4.0 and 12.0 kV for various nozzles in order to influence the mode of electrospraying in a qualitative manner.
(ii) Type of nozzle; the nozzle with a tilted outlet (apex angle 30°) was used for the deposition of coatings onto cpTi substrates.
(iii) Relative precursor concentrations, i.e. the Ca/P ratio; the molar Ca/P ratio of the precursor solution will be reflected in the composition of the deposited coating, for instance corresponding with the Ca/P ratio for hydroxyapatite the Ca/P ratio of the precursor solution is 1.67. For instance a Ca/P ratio of 1.80 may correspond with the Ca/P ratio of a B-type CAp.
(iv) Absolute precursor concentrations; for instance the Ca²⁺ concentration can be varied between 0.001 M and 0.025 M.
(v) Solvent composition; for instance an alcohol having a low boiling point (ethanol; (ET)) or an alcohol having a high boiling point (butyl carbitol (BC)) or mixtures thereof can be used.
(vi) Solution additives; for instance water can be added (1-5 vol%) or HNO₃ (65%) can be added (0.25 - 1 vol%) to the precursor solutions.
(vii) Flow rate Q [ml/h]; the flow rate of the precursor solution can be varied for instance from 0.2 to 5.0 ml/h.
(viii) Substrate temperature T [°C]; the substrate temperature can be varied and the substrate can be heated to a temperature for instance between 250 °C and 450 °C.
(ix) Nozzle-to-substrate distance d [cm]; d can be varied for instance from 2.5 cm to 3.5 cm.
(x) Deposition time; the deposition time can be varied for instance from 7.5 min to 2h.
(xi) Substrates; depending on the use material and form of the substrate can vary.

### Deposition of ESD-coatings with defined morphologies

Reticular, spongy coating morphologies form under wet conditions with only moderate rates of precipitation and solvent evaporation, whereas the broccoli-like coatings will form as a result of considerable solvent evaporation and solute precipitation.
In order to synthesize spongy coating morphologies, relatively high flow rates were chosen, whereas the substrate temperature and nozzle-to-substrate distances were set at relatively low values. BC was chosen as the appropriate solvent for its high boiling point, corresponding to a reduced extent of solvent evaporation. In the search of this unique coating morphology, the following deposition series were performed in order to investigate the influence of specific process parameters on the morphology of the produced coatings. In each series, all other deposition parameters were held constant.
- The flow rate Q: 0.5 - 1.0 - 1.5 - 2.0 - 2.5 - 3.0 - 4.0 - 5.0 ml/h
- Deposition time t: 7.5 min - 15 min - 22.5 min - 30 min - 1h - 2h
- Substrate temperature T: 300 - 325 - 350 °C
- Substrate topography: machined and polished cp-Ti substrates
- HNO₃ addition: 0 vol% and 0.5 vol% HNO₃ (65%)

For formation of the broccoli-like coating morphology, elevated substrate temperatures and large nozzle-to-substrate distances were used. Moreover, pure ethanol was used as solvent for its low boiling point. However, it was also tried to synthesize broccoli-like morphologies with pure BC as a solvent.
All coating morphologies were observed using a scanning electron microscope (SEM, JEOL JSM-35).

### Structure of the ESD-coatings: heat treatments

In order to obtain insight in the crystal structure and molecular structure of the deposited coatings, several deposited coatings were subjected to heat treatments. With conventional heat treatments in an electric furnace at elevated temperatures and long duration, the underlying Ti-substrate oxidized severely, thereby inhibiting a proper characterization of the deposited CaP coatings. Therefore, rapid heat treatments of 5 - 30 seconds of various intensities and durations were performed by infrared radiation (Quad Ellipse Chamber, Model E4-10-P, Research Inc.). Heat treatments at low temperatures and short duration were shown to be able to reduce the oxidation of titanium and diffusion of elements that influence the bond between the films and titanium substrate.
Infrared radiation was carried out under atmospheric conditions. The maximum temperature of the rapid heat treatments was measured with a Pt-PtRh thermocouple close to the specimen. Both the thermocouple and the coated substrate were placed onto flat, heat-resistant ceramic plates. Prior to each rapid heat treatment, the coated substrates were pre-heated with a low IR radiation intensity up to ± 160 °C. Upon reaching this temperature, the actual heat treatment was carried out.
Although this method is a very easy and fast method to perform heat treatments upon CaP-coated metal substrates, it has to be stated that rapid heating is not an equilibrium heat treatment. Long-term diffusion effects like in conventional sintering treatments are absent due to the very short effective heating time. Also, the observed temperature as measured by the thermocouple close to the coated substrates does not represent the actual temperature in the ceramic coating. Therefore, the measured temperatures were considered as indications instead of exactly measured temperatures.
After rapid heat-treatments, the coated substrates were subjected to X-Ray Diffraction (XRD) and Fourier-Transform Infrared Spectrometry (FTIR).

### Composition of the ESD-coatings

In order to characterize the composition of the deposited ESD-coatings, some samples were subjected to Rutherford Backscattering Spectrometry (RBS) and Energy Dispersive Spectroscopy (EDS).
RBS is the only nondestructive techniqe that provides simultaneous depth and composition information. This technique is quantitatively precise to within an atomic percent from first principles and requires no use of composition standards. The lateral spatial resolution of the region over which analysis can be performed is ± 1mm. The analysis depth is typically a few µm. If RBS of CaP films yields a spectrum with sharp Ca and P steps, Ca/P ratios can be calculated.
EDS was also used to characterize the elemental composition and Ca/P ratios of the deposited coatings. Pure, stoichiometric hydroxyapatite with a known Ca/P ratio of 1.67 was used as a reference material. However, EDS gives only semi-quantitative information and calculated Ca/P ratios should only be considered as an indication of the Ca/P ratio instead of an exact value.

### Characterization methods

### Scanning Electron Microscopy/Energy Dispersive Spectroscopy

Scanning Electron Microscopy (SEM) was carried out using a JEOL JSM-35 microscope at accelerating voltages between 10 - 20 kV. All as-prepared ESD coatings were examined without deposition of an additional gold coating, as their porous morphologies allowed enough transport of electrons through the coating to avoid charging of the samples. In contrast, all sputter-coated samples and cell-cultured samples were sputter-coated with gold.
The above described scanning electron microsope was equipped with an energy-dispersive X-ray microanalyzer (Link ISI, Oxford Instruments Ltd.). EDS was carried out at a magnification of 500x at an accelerating voltage of 15 kV. Stoichiometric hydroxyapatite was used as a reference for the determination of CaP ratios.

### X-Ray Diffraction

CaP powders, as prepared by precipitation of precursors in BC, and all CaP coatings were subjected to X-Ray Diffraction (XRD) analysis on a thin-film Philips X-Ray Diffractometer using CuKα-radiation (PW 3710, 40 kV, 40 mA).
Powders were analyzed in a θ-2θ mode at a scanning range from 2θ = 20° to 2θ = 50° with a step-size of 0.02 ° 2θ, a scanning speed of 0.01 °2θ/s and a sample time of 2 s/step.
Coatings were analyzed by fixing the sample to a position of 2.5° and scanning the detector between 20° and 50 ° at the same measuring conditions as described above.
Cell-cultured samples were also analyzed by thin-film XRD, but in a shorter scanning range from 25° to 40 °2θ.

### Fourier-Transform Infrared Spectrometry

The infrared spectra of the films on the substrates were obtained by reflection Fourier-Transform Infrared Spectrometry (FTIR, Perkin-Elmer), since the infrared radiation cannot pass through the titanium substrate. Spectra were obtained by averaging 30 scans. In order to obtain the FTIR-spectra of the precipitated powders, a KBr method was applied: sample/KBr weight ratio was 1/250.

### Rutherford Backscattering Spectrometry

Standard ⁴He⁺ Rutherford Backscattering Spectrometry (RBS) at 2 or 2.4 MeV at scattering angles of 170° and 120° was used to determine the composition of the coatings and to calculate Ca/P ratios with the computer program RUMP.

### Biological evaluation of CaP ESD-coatings Uncoated titanium substrates

Unpolished, machined commercially pure cp-Ti substrates (⌀ 12mm, 1.5 mm thickness) were cleaned ultrasonically in acetone (15 min) and ethanol (15min). These discs were provided with a CaP coating using the ESD technique.

### ESD CaP coatings

The process conditions for the deposition of the ESD-derived sponge- and broccoli-coatings were as follows:

| ESD parameters for deposition of sponge- and broccoli-coatings for use in cell culture | | |
|---|---|---|
| | **ESD-sponge** | **ESD-broccoli** |
| Ca²⁺ concentration [M] | 0.005 | 0.005 |
| H₃PO₄ concentration [M] | 0.003 | 0.003 |
| Solvent composition | BC | BC |
| Time [min] | 45 min | 45 min |
| Temperature [°C] | 300 °C | 450 °C |
| Flow rate [ml/h] | 2.0 | 0.5 |
| Nozzle-to-substrate distance [cm] | 2.5 | 3.5 |
| Nozzle apex angle [°] | 30 | 30 |
| | | 15 |
| Mode of electrospraying | duo-jet | duo-jet/cone-jet |
| Applied potential difference [kV] | 6.5 - 8.0 | 6.5 - 9.0 |

For both ESD-coating morphologies, butyl carbitol was used although it is assumed that ethanol would give better results for the synthesis of broccoli-like morphologies due to its lower boiling temperature. However, in order to keep the chemical composition of both broccoli- and sponge-coatings as much the same butyl carbitol was also used for the synthesis of broccoli-like coatings.
During deposition of the series of broccoli-coatings, the nozzle with an apex angle of 30° clogged due to the solute precipitate, which made it necessary to use a sharper nozzle with an apex angle of 15°. Although the nozzle geometry may affect the ESD process for a broccoli-like coating morphology, the deposition temperature is supposed to dominate over other deposition parameters.

After deposition, the ESD-coatings were heat-treated by IR-radiation as described before. All ESD coatings were at least subjected to a single rapid heat treatment of ± 500 °C to remove nitrate from the coatings. Subsequently, half of the denitrated samples were heat treated further in two steps of ± 570 and ± 600 °C, respectively. These latter coatings were labelled "crystalline" coatings (n=1). The coatings that were only denitrated at ± 500 °C were designated "amorphous" (n=1). X-Ray Diffraction and Fourier-Transform Infrared Spectrometry (FTIR) was carried out on both amorphous and crystalline broccoli- and sponge-coatings to determine their crystallographic and molecular structure.

Before use in the cell culture experiments, all coated and uncoated samples were sterilized in 70% ethanol, washed twice in phosphate-buffered saline (PBS), placed at the bottom of each well of a 24-well plate (Greiner B.V.) and incubated in culture medium at 37 °C for 30 minutes.

### Cell isolation and culture

For the biological evaluation of the test materials a rat bone marrow (RBM) cell culture technique was used as described by Maniatopoulos et al. Cell Tiss. Res., 254, 1988, 317-330. Briefly, both femora of young adult mate Wistar rats (weight 100-120 g, age 40-43 days) were removed and washed four times with α-Minimal Essential Medium (MEM, Gibco, Life Technologies), containing 0.5 mg/ml gentamycin (Gibco) and 3.0 µg/ml fungizone (Gibco). Afterwards, the epihyses were cut off and the diaphyses flushed out, using α-MEM supplemented with 10% fetal calf serum (FCS, Gibco), 50 µg/ml ascorbic acid (Sigma), 10 mM Na β-glycerophosphate (Sigma), 10⁻⁸ M dexamethasone (Sigma) and 50 µg/ml gentamycin (Gibco). Finaly, cultures were incubated in a humidified atmosphere of 5% air, 5% CO₂ at 37 °C. After 7 days of primary culture, cells were detached using trypsin/EDTA (0.25% w/v trypsin/ 0.02% EDTA, ethylenediaminetetraacetic acid) and the cells were suspended in the supplemented culture medium as described above.

### Cell morphology assay

RBM cell suspension (1ml per well, containing 4-10⁴ cells) was added to the test substrates. The cultures were incubated for 2, 7 and 14 days at 37 °C in 5% CO₂-air atmosphere. At incubation day 1, 3, 6, 8, 10 and 13, the culture medium was refreshed. At the end of the various incubation periods (2, 7, and 14 days), the non-attached cells were removed by PBS rinses. The attached cells were fixed with 2% v/v glutaraldehyde in 0.1 M sodium cacodylate buffered solution for 15 minutes, rinsed twice in 0.1 M cacodylate buffered solution, followed by dehydration through a graded series of ethanol. Subsequently, the specimens were dried by tetramethylsilane (Merck). Finally, after sputter-coating with gold, the samples were examined using a scanning electron microscope (SEM, JEOL JSM-35) at an accelerating voltage of 15 kV. X-Ray Diffraction was carried out on the same samples to investigate the crystallographic properties of the coatings after culture.

### Results

### Range of deposition parameters

A qualitative description will be given of the influence of the various deposition parameters, that were mentioned hereinabove, on the spraying characteristics during coating formation with ESD.
(i) Until electric field strengths of about 2.4 kV/cm, the cone-jet was observed. The maximum flow rate in the cone-jet mode for this precursor liquid was ±0.5 ml/h. At higher flow rates, the excess of liquid simply overflowed the nozzle. This low maximum flow rate corresponded to low deposition rates and very long deposition times. At higher voltages of more than ± 2.4 kV/cm, the duo-jet mode of electrospraying was observed. In this electrospraying mode, much higher flow rates of more than 5.0 ml/h could be obtained due to the higher acceleration of the liquid in the two cones and jets. Therefore, the duo-jet spraying mode was chosen in order to deposit the Ca/P coatings.
   Several duo-jet modes were investigated in their relation to the homogeneity of the deposited coatings. It was observed that the homogeneity of the coatings decreased as the stability of the duo-jet increased. Mixed cone- and duo-jet modes, shifting between both modes with a high frequency, yielded homogeneous coatings, whereas the coatings became heterogeneously divided into two parts when a more stable duo-jet was used. This could be attributed to the fact that the two positively charged cones in a stable duo-jet repel each other laterally. As a consequence, a separation between the two generated positively charged sprays exists. If the substrate was positioned at the exact location of this separation zone, which exhibits a very low spray droplet density, deposition rate is locally very low, which results into heterogeneous coatings. On the contrary, the horizontal movements of the shifting cones in the mixed cone/duo-jet modes gave rise to a mixed, polydisperse spray. This resulted into homogeneous spray droplet densities and coatings.
(ii) Nozzles with tilted outlets (apex angle 15° or 30°) were used.
(iii) Relative Ca/P concentrations had an indirect influence on the spraying characteristics. Solutions with a Ca/P ratio of 1.80 (0.05 M Ca(NO₃)₂.4H₂O + 0.028 M H₃PO₄ in BC) did not precipitate within 2 hours. On the contrary, the stoichiometric Ca/P solution ratio of 1.67 (0.05 M Ca(NO₃)₂.4H₂O + 0.03 M H₃PO₄ in BC) contained a slightly higher H₃PO₄ concentration and started to precipitate after ±30 minutes. Since most deposition times were longer than 30 minutes, some precipitation during the deposition could not be avoided. Precipitating precursor solutions show a tendency towards higher spraying modes (duo and/or trio-jets depending on the degree of precipitation). Therefore, the applied potential difference had to be reduced during deposition in order to stay in the same duo-jet spraying mode.
   During precipitation, free ions in the liquid precipitate into neutral compounds. Consequently, the conductivity of the solution decreases and the jet diameter increases, whereas the current and surface charge density decrease. As a result, the kinetic energy of the jet decreases. Therefore, less electric power (i.e. lower potential differences) are needed to stay in the same duo-jet spraying mode. In view of this, the observed tendency towards higher spraying modes is a logical consequence, since the applied potential difference was kept at a constant value. With increasing precipitation and decreasing conductivity of the precursor solutions, the applied potential became actually too high for the duo-jet mode.
(iv) Absolute precursor concentration directly influenced the spraying characteristics in the same way as described above. Decreasing the Ca²⁺ precursor concentration from 0.005 to 0.0025 M Ca²⁺ in BC resulted into a longer (duo)-jet length and a reduced potential difference needed to maintain the duo-jet mode. Again, decreasing the precursor concentration corresponds to a lower conductivity and consequently a lower kinetic energy of the jet. Therefore, less electric power is needed to stay in the same duo-jet spraying mode. The longer jet length was caused by the reduced surface charge density in the jet that was accompanied by a reduced whipping motion of the jet.
(v) Using butyl carbitol as solvent, very clear and stable sprays were obtained which reflects suitable spraying properties regarding the conductivity and surface tension. The high boiling temperature of BC enabled a clear visualization of the spraying process, as the droplet evaporation was inconsiderable. On the other hand, the conductivity of the precursor solutions in ethanol was approximately two orders of magnitude lower as compared to butyl carbitol as a solvent. Consequently, the observed maximum flow rate in ethanol-precursor solution was much lower and deposition times were longer. Therefore, BC was chosen as standard solvent. However, the lower dielectric constant of BC as compared to ethanol makes precipitation of precursor in BC more easy than in ethanol.
(vi) The addition of water was immediately followed by severe precipitation that decreased conductivity. Consequently, the jet length increased and a tendency towards the trio-jet mode was observed, as mentioned already above. The addition of HNO₃ resulted into an enhanced conductivity. As a result, the jet length was reduced due to the decreased whipping motion of the jet. Moreover, more electric power was needed in order to maintain a stable duo-jet mode due to the increased conductivity. This results into a large surface charge density and a higher kinetic energy of the jet. Accordingly, higher potential differences had to be applied.
(vii) Increasing the flow rate was accompanied by a simultaneous increase in potential difference needed to avoid overflow of the precursor liquid. When the flow rate increases, the current through the cone increases also. This effect occurs because a higher flow rate requires more electric power to accelerate the liquid in the jet. Increasing the current also leads to increased homogeneity of the spray and coating. A possible explanation for this phenomenon is the increased whipping motion that is the result of a higher current. Whipping corresponds to mixing of the spray droplets.
(viii) Substrate temperatures had a slight influence on spraying characteristics. Using ethanol as a solvent at high substrate temperatures and low nozzle-to-substrate distances, no stable spray could be maintained. Boiling ethanol in the liquid cone destabilized the spray formation by formation of air bubbles.A second but small influence on the spraying behavior was the observation that clogging of the nozzle became a problem at higher temperatures due to solute precipitation inside the nozzle. Precipitation seemed to be triggered by the high temperature of the nozzle. This is in line with equation 11, which theoretically predicts an increased nucleation rate under the influence of a high precipitation temperature. At high substrate temperatures, the temperature of the metal nozzle is also relatively high due to the excellent thermal conductivity of metals in general.
(ix) With respect to the influence of the temperature on clogging behavior of the nozzle, a small nozzle-to-substrate distance also resulted into increased clogging of the nozzles. Furthermore, boiling of ethanol in the liquid cone was more pronounced at shorter distances.
(x) Deposition times influenced the spraying characteristics indirectly. Long deposition times of more than 45 minutes unavoidably led to premature precipitation of the precursor solution during deposition when using supersaturated solutions with a Ca/P ratio of 1.67.

### Deposition of ESD-coatings with defined morphologies

### Process conditions

Optimised process conditions for the deposition of the ESD-derived sponge- and broccoli-coatings were as follows:

| ESD parameters for deposition of sponge- and broccoli-coatings for use in cell culture | | |
|---|---|---|
| | **ESD-sponge** | **ESD-broccoli** |
| Ca²⁺ concentration [M] | 0.005 | 0.01 |
| H₃PO₄ concentration [M] | 0.003 | 0.06 |
| Solvent composition | butyl carbitol | ethanol |
| Temperature [°C] | 300 °C | 450 °C |
| Flow rate [ml/h] | 2.0 | 0.5 |
| Deposition time | 1 hour | 2 hours |
| Nozzle-to-substrate distance [cm] | 2.5 | 3.5 |
| Nozzle apex angle [°] | 30 | 30 |
| Mode of electrospraying | duo-jet | cone-jet |
| Applied potential difference [kV] | 6.5 - 8.0 | 6.5 - 8.0 |

Sponge coatings (Figure 2) were formed using relatively low concentrations of precursors, also because of the low solubility of the precursors in the apolar solvent. Moreover, low precursor concentrations correspond to low conductivities and large droplet sizes. The duo-jet mode of electrospraying enabled the use of a high liquid flow rate, which is thought to be necessary to form the reticular, interconnected structures. Further, the substrate temperature of 300 °C is just 69 °C higher than the boiling point of butyl carbitol. As a consequence, solvent evaporation will be inconsiderable since the very high droplet velocities of several tenths of m/s correspond to flight times in the order of 0.1-1 ms.

On the contrary, broccoli coatings (Figure 3) can be formed using higher precursor concentrations in ethanol, which correspond to higher conductivities and lower droplet sizes. These smaller droplets are subject to enhanced solvent evaporation due to a higher substrate temperature, a larger nozzle-to-substrate-distance and a solvent that evaporates much faster. Also, the lower electric field strength of the cone-jet results into shorter droplet flight times. The maximum flow rate of ethanol solutions in the cone-jet mode was quite low. Moreover, deposition efficiency was less due to the large nozzle-to-substrate distance. Therefore, longer deposition times were needed in order to obtain coatings of approximately equal thickness as compared to sponge coatings.

### Influence of deposition parameters on sponge morphologies Flow rate

The influence of increasing liquid flow rate on the morphology of ESD-derived sponge coatings can be summarised as follows. Generally, the pore size decreased, whereas the pore size wall thickness increased due to an increasing amount of solute deposition per unit of time.

### Deposition time

The initial characteristics of the sponge coating morphology were already recognizable after 15 minutes of deposition time.

### Deposition temperature

Going from a substrate temperature of 300°C to 350°C the sponge coating morphology was transformed into a less interconnected, broccoli-like morphology in an initial development stage. This indicates that the substrate temperature is a critical parameter controlling the formation of the sponge coating.

### Addition of HNO₃

The addition of 0.5 vol% HNO₃ resulted into the formation of broccoli-like coatings instead of sponge morphologies. This can be attributed to the increased conductivity of the precursor solutions, that corresponds to smaller droplet sizes. Consequently, droplets arrive at the substrates in almost dry condition.

### Substrate topography

Figure 2 shows the influence of substrate topography on the morphology of ESD-derived sponge coatings for a polished cp-Ti substrate. Figure 2 shows that the sponge coating morphology develops onto polished substrates. The mechanism of formation of sponge morphologies appears not to be dependent on the presence of small grooves, although orientation of the sponge morphology was guided by small microgrooves due to machining. Figure 4 shows the influence of substrate topography on sponge coating morphology for a machined cp-Ti substrate. In Figure 4 the large machining grooves are clearly recognizable that are reflected in the coating morphology as a result of preferential landing of the charged spray droplets onto regions of positive curvature. Sponge morphologies became aligned along small machining grooves.

### Influence of substrate temperature on coating structure

Sponge coatings deposited at substrate temperatures below 350 °C were amorphous with regard to CaP compounds without any (rapid) heat treatment. On the contrary, as-deposited broccoli-coatings using butyl carbitol as solvent revealed already a broad apatite peak indicating initial crystallization due to the high substrate temperature of 450 °C. Moreover, no nitrate absorptions were found by means of FTIR-spectroscopy.

### Influence of the addition of water on coating structure

Relevant deposition conditions to investigate the influence of the addition of water on coating structure are as follows.

| ESD parameters for the deposition of apatite ESD-coating using water-supplemented precursor solutions. | |
|---|---|
| | **H**_{**2**}**O coating** |
| Crystal phases [XRD] | calcite + carbonated apatite |
| Ca²⁺ concentration [M] | 0.005 |
| H₃PO₄ concentration [M] | 0.0028 |
| Ca/P solution ratio | 1.80 |
| H₂O addition [vol%] | 4.0 |
| Solvent composition | butyl carbitol |
| Time [h] | 2 |
| Heat treatment | IR rapid heating until 740 °C |

With respect to FTIR-spectroscopy, it can be concluded that phosphate and carbonate ions are incorporated in a different manner as compared to the crystalline carbonate apatite coating without water added in the precursor solution.

Regarding XRD, the addition of 4 vol% water resulted into the formation of CaCO₃ in the calcite modification at 500 °C. Moreover, crystallinity of the apatite phase was reduced as compared to the crystalline carbonate apatite coating.

### Influence of precipitation on coating structure

Relevant deposition conditions of an ESD-coating that was deposited using precipitating precursor solutions without any water added were as follows.

| ESD parameters for the deposition of apatite ESD-coating using precipitating precursor solutions. | |
|---|---|
| | **Precipitation coating** |
| Crystal phases [XRD] | calcite |
| Ca²⁺ concentration [M] | 0.005 |
| H₃PO₄ concentration [M] | 0.0028 |
| Ca/P solution ratio | 1.80 |
| Solvent composition | butyl carbitol |
| Time [h] | 1 ½h |
| Heat treatment | IR rapid heating until 740 °C |

Calcite was already being formed on the as-deposited coating. With increasing heating temperature, calcite was decomposed. Precipitation during deposition has a strong influence on coating structure.

### Biological evaluation of CaP ESD-coatings

### Cell responses: 2 days in culture

RBM cells attached to broccoli-type ESD-coatings. Besides normal attached cells, agglomerations of RBM-cells into flattened cell layers were observed for all broccoli-coatings.

RBM cells attached to sponge-type ESD coatings. The cells preferentially aligned along the sponge structure. The alignment of the sponge structure results from the substrate topography of the machined commercially pure Ti. Long, elongated cells with a length of up to 100 µm and long filopodia were frequently observed. Moreover, cytoplasmatic extensions of the attached RBM-cells seemed to fuse intimately with the pore walls of the sponge morphology, see Fig. 5.

### Cell responses: 7 days in vitro

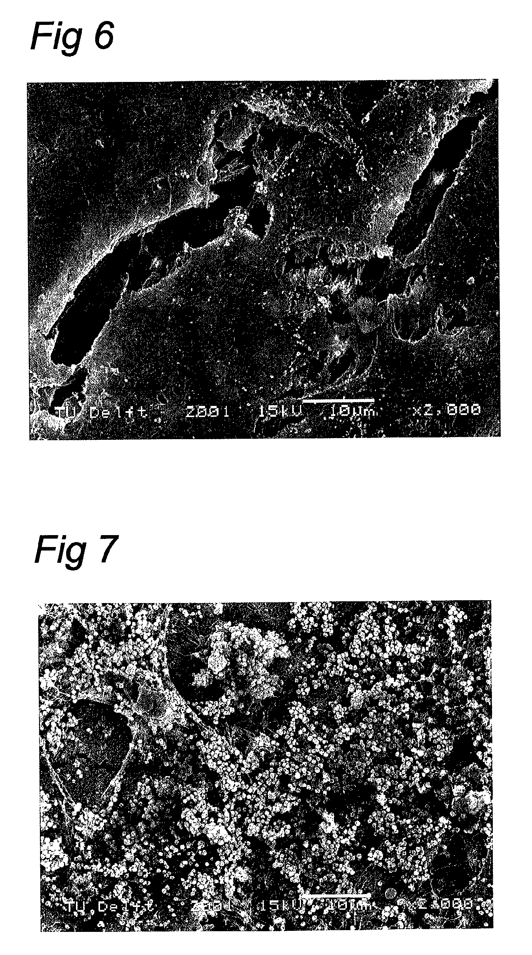

After 7 days a homogeneous and thick multilayer of osteoblast-like cells on a carbonate containing ESD coatings with a broccoli-like morphology was observed, see Fig. 6. Some collagen bundles and globular accretions were observed, indicating that the formation of a calcified extracellular matrix was initiated.

Figure 8 shows abundant extracellular matrix production onto ESD-sponge coatings with large amounts of collagen fibres and globular accretions. The size of the calcifications was larger in comparison to broccoli-ESD coatings, which indicates an increased rate of mineralization. Coating crystallinity is of influence on coating stability and subsequent cellular response under in vitro circumstances. A less crystalline sponge coating, due to the heat treatment, showed after 7 days of incubation in cell culture medium a thin, heterogeneous cell layer covering the remains of the sponge coating.

### Cell responses: 14 days in vitro

A thick layer of rich collageneous matrix secreted by osteoblast-like cells and associated with globular calcified accretions is observed on ESD-broccoli coatings.

Abundant production of collagen bundles and globular calcifications onto ESD-sponge coatings after 14 days in culture is observed.

In conclusion, it was shown by means of rat bone marrow cell cultures that ESD-CaP coatings possess the capacity to activate the differentiation and expression of osteogenic cells. SEM investigations indicated a coating morphology dictated type of cellular response for the ESD-derived coatings after 2 and 7 days in culture. Also crystal structure and chemical composition may play a role.

## Claims

1. Method for depositing a coating onto an implant for implantation in bone comprising the steps of forcing a precursor solution through a capillary which is subjected to an electrical field and placing the implant in the area of the spray that leaves the outlet of the capillary.

2. Method according to claim 1 in which the precursor solution comprises calcium and phosphate.

3. Method according to claim 2 in which the molar ratio of calcium to phosphate in the precursor solution is in the range of about 0.5 to about 2, preferably is in the range of about 1.5 to about 1.8, more preferably is in the range of about 1.67 to about 1.8, even more preferably is about 1.67.

4. Method according to claim 2 or 3 in which the precursor solution comprises calcium nitrate and phosphoric acid.

5. Method according to any of the preceding claims in which the solvent in the precursor solution is an alcohol, preferably ethanol or butyl carbitol or a mixture thereof.

6. Method according to any of the preceding claims in which the precursor solution comprises additives such as water, preferably 1-5 vol%, or nitric acid, preferably 0.25-1 vol% HNO₃ 65%.

7. Method according to any of the preceding claims in which the precursor solution further comprises trace elements to support cell growth such as bone growth stimulating proteins.

8. Method according to any of the preceding claims in which the precursor solution further comprises glass forming components such as tetramethyl ortho-silicate and sodium hydroxide.

9. Method according to any of the preceding claims in which at least the surface of the implant comprises niobium, tantalum, aluminium oxide, cobalt-chromium alloys, (stainless) steel and in particular titanium and titanium alloys.

10. Method according to any of the preceding claims in which the implant is heated, preferably to a temperature in the range of 250°C to 450°C.

11. Method according to any of the preceding claims which further comprises the step of heating the coated implant to a temperature n the range of 500-1250°C, preferably by infrared radiation for 5-30 s.

12. Method according to any of the preceding claims in which the flow rate that is used to force the precursor solution through the capillary is in the range of 0.2-5 ml/h, preferably in the range of 1-3 ml/h and the electrical field to which the capillary is subjected is in the range of 4-12 kV, preferably in the range of 6-9 kV.

13. Implant for implantation in bone provided with a coating obtainable by the method according to any of the preceding claims.

14. Implant according to claim 13 said implant having a porous coating comprising calcium and phosphate.

15. Implant according to claim 14 in which the porous coating has pores in the range of 0,1-25 µm, preferably in the range of 0,5-15 µm, more preferably in the range of 0.8-10 µm.

16. Dental implant according to any of claims 13-16.
